(19) **Europäisches Patentamt**

**European Patent Office**

**Office européen des brevets**

(11) **EP 1 720 008 A1**

(12) **EUROPEAN PATENT APPLICATION**
published in accordance with Art. 158(3) EPC

(43) Date of publication:
**08.11.2006 Bulletin 2006/45**

(51) Int Cl.:
***G01N 27/12*** *(2006.01)*

(21) Application number: **05719728.7**

(22) Date of filing: **23.02.2005**

(86) International application number:
**PCT/JP2005/003411**

(87) International publication number:
**WO 2005/080952 (01.09.2005 Gazette 2005/35)**

(84) Designated Contracting States:
**DE FR GB**

(30) Priority: **24.02.2004 JP 2004047681**

(71) Applicant: **MIKUNI CORPORATION**
**Chiyoda-ku,**
**Tokyo 101-0021 (JP)**

(72) Inventors:
• **FUKUI, Katsuhiko,**
**Mikuni Corporation Morioka Branch**
**Iwate-gun, Iwate 020-0188 (JP)**

• **HIRAYAMA, Toshinori,**
**Mikuni Corporation**
**Takizawa,**
**Takizawa-mura, Iwate-gun, Iwate (JP)**

(74) Representative: **Ziebig, Marlene et al**
**Anwaltskanzlei**
**Gulde Hengelhaupt Ziebig & Schneider**
**Wallstrasse 58/59**
**10179 Berlin (DE)**

(54) **CARBON MONOXIDE SENSOR AND METHOD FOR SENSING CARBON MONOXIDE**

(57) The present invention discloses a carbon monoxide sensor comprising
a housing having a to-be-analyzed-gas inlet fitted with a filter for removal of nitrogen oxides and sulfur oxides, and a detection element provided in the housing, which comprises a P type metal oxide semiconductor composed mainly of copper oxide,
wherein the filter is made of an inorganic material. The inorganic material constituting the filter is preferably a zeolite having a component ratio $SiO_2/Al_2O_3$ (mole basis) of 5 to 510.

Fig. 1

(a)

(b)

EP 1 720 008 A1

## Description

Technical Field

The present invention relates to a carbon monoxide sensor for detecting carbon monoxide (CO gas) contained in a to-be-analyzed gas at good selectivity, as well as to a method for detection of carbon monoxide contained in a to-be-analyzed gas, using the carbon monoxide sensor.

Background Art

It has been conducted to detect a very small amount of gas contained in the air, using a gas sensor having a ceramic semiconductor as a detection element. As the semiconductor used as a detection element, N type ceramic semiconductors are known well. There is also known a carbon monoxide sensor using a P type ceramic semiconductor as a detection element. The P type ceramic semiconductor is constituted by a metal oxide composed mainly of copper oxide ($CuO$) (Claim 1 of Japanese Patent No. 3333257).

The sensitivity of the above detection element composed mainly of copper oxide, to carbon monoxide varies depending upon the oxide components contained in a to-be-analyzed gas, such as nitrogen oxides (NOx), sulfur oxides (SOx) and the like. Therefore, in order to detect the carbon monoxide contained in a to-be-analyzed gas, selectively and at good accuracy, it is necessary to beforehand remove the oxide components contained in the to-be-analyzed gas, using a filter or the like and then detect the carbon monoxide.

As the carbon monoxide sensor provided with a filter for removal of NOx and SOx, there is a sensor wherein an active carbon filter is provided in a to-be-analyzed gas passage, which is communicated with a detection element (paragraph No. 0011 of JP-A-2000-338072). In this literature, it is described that, by providing the active carbon filter, the carbon monoxide contained in the to-be-analyzed gas can be detected selectively and stably.

Disclosure of the Invention

The present inventors conducted tests for detection of carbon monoxide systematically and repeatedly, using a number of gas sensors each provided with an active carbon filter. In the tests, there occurred disappearance or contraction of active carbon filter, and the present inventors frequently encountered a phenomenon in which the sensitivity of carbon monoxide detection became unstable or dropped. This phenomenon is considered to appear because, in measurement of carbon monoxide, the active carbon filter (which is an organic material) is oxidized by the high-temperature combustion gas (to-be-analyzed gas) discharged from, for example, a gas appliance and resultantly thins and vanishes.

The present invention aims at providing a carbon monoxide sensor and a method for detection of carbon monoxide, both capable of detecting, selectively and stably, the carbon monoxide contained in a to-be-analyzed gas such as high-temperature combustion gas containing NOx and SOx, or the like.

The present inventors made a study in order to achieve the above aim. As a result, it was found that a filter made of an inorganic material, particularly a zeolite filter is suitable as a filter for removal of NOx and SOx. The finding has led to the completion of the present invention.

[1] A carbon monoxide sensor comprising
a housing having a to-be-analyzed-gas inlet fitted with a filter for removal of nitrogen oxides and sulfur oxides, and
a detection element provided in the housing, which comprises a P type metal oxide semiconductor composed mainly of copper oxide,
wherein the filter is made of an inorganic material.
[2] A carbon monoxide sensor according to [1], wherein the inorganic material is a zeolite having a compositional ratio $SiO_2/Al_2O_3$ (mole basis) of 5 to 510.
[3] A carbon monoxide sensor according to [1], wherein the filter is made of a non-fired zeolite.
[4] A carbon monoxide sensor according to [1], wherein the filter is obtained by press molding of a non-fired zeolite.
[5] A carbon monoxide sensor according to [1], wherein the filter is obtained by firing of a zeolite having an average particle diameter of 150 $\mu$m or less.
[6] A carbon monoxide sensor according to [1], wherein the filter is formed in a disc shape.
[7] A carbon monoxide sensor according to [1], wherein the filter has a water-proof agent coated thereon.
[8] A carbon monoxide sensor according to [1], wherein the filter has a laminated structure of a zeolite layer and an active carbon layer and is fitted to the to-be-analyzed-gas inlet with the zeolite layer being directed toward the outside of the housing.
[9] A carbon monoxide sensor according to [1], wherein the filter has an inorganic cushion material layer on at least one side of the filter.

[10] A carbon monoxide sensor according to [9], wherein the inorganic cushion material layer is made of an alumina fiber.

[11] A carbon monoxide sensor according to [9], wherein the cushion material layer has a thickness of 0.4 to 1 mm.

[12] A method for detection of carbon monoxide, which comprises detecting a to-be-analyzed gas of 60 to 250°C using a carbon monoxide sensor set forth in Claim 1.

The carbon monoxide sensor of the present invention is provided with a non-oxidizable inorganic filter and therefore can detect the carbon monoxide present in a high-temperature, to-be-analyzed gas containing NOx and SOx, at good selectivity over a long period of time. The carbon monoxide sensor of the present invention is suitable as a sensor used in a gas alarm device in order to detect the carbon monoxide present in a combustion gas which is discharged from a combustion gas apparatus, a heating apparatus, a boiler or the like.

Brief Description of the Drawings

Fig. 1 is a plan view (a) showing an example of the carbon monoxide gas sensor of the present invention and a sectional view (b) thereof.

Fig. 2 is a sectional view showing a constitution of the filter fitted to the carbon monoxide gas sensor of the present invention.

Fig. 3 is a sectional view showing other constitution of the filter fitted to the carbon monoxide gas sensor of the present invention.

Fig. 4 is a graph showing the changes with time of the sensor resistances measured in Example 1 and Comparative Example 1.

Fig. 5 is a graph showing the effects of $NO_2$ on the outputs of the sensors of Example 1 and Comparative Example 1.

Fig. 6 is a graph showing the effect of temperature on the output of the sensor of Example 2.

Fig. 7 is a graph showing the changes with time of the sensor resistances measured in Example 3 and Comparative Example 2.

Fig. 8 is a graph showing the effects of $NO_2$ on the outputs S of the sensors of Example 3 and Comparative Example 2.

Fig. 9 is a graph showing the effect of $SO_2$ on the resistance of the sensor of Example 1.

Numeral 101 is a filter; numeral 2 is a to-be-analyzed-gas inlet; numeral 3 is an upper wall; numeral 4 is an upper housing; numeral 5 is a bottom housing; numeral 6 is a connecting portion; numeral 7 is a filter-supporting portion; numeral 8 is a housing; numeral 10 is a detection portion; numeral 12 is a detection element; numeral 14 is a ceramic substrate; numerals 16 and 18 are each a support for detection portion; numerals 20 and 26 are each a zeolite layer; numerals 22 and 24 are each an inorganic cushion material layer; numeral 28 is an active carbon layer; and numeral 100 is a carbon monoxide sensor.

Best Mode for Carrying Out the Invention

An embodiment of the carbon monoxide sensor of the present invention is described in detail below with referring to the Drawings.

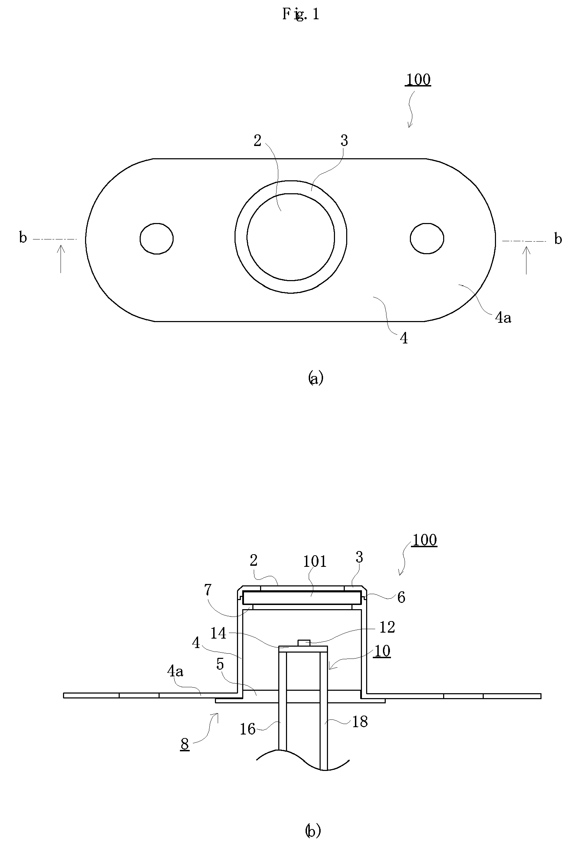

Fig. 1(a) is a plan view showing an example of the carbon monoxide sensor of the present invention. Fig. 1(b) is a sectional view taken at the b-b line of Fig. 1(a).

In Fig. 1, 100 is a carbon monoxide sensor of the present invention.

An upper housing 4 has a cylindrical shape wherein the outside and the inside are separated air-tightly, and the lower end of the upper housing 4 has a base portion 4a which protrudes in the radial direction of upper housing.

At the inner wall of the upper housing 4 is formed, along the inner circumference, a ring-shaped, filter-supporting portion 7. Further, at the upper end of the upper housing 4 is formed an upper wall 3. In the upper wall 3 is formed a to-be-analyzed-gas inlet 2 for introducing a to-be-analyzed gas into the upper housing 4. Incidentally, the upper wall 3 can be detachably fitted to the upper housing 4 at a connecting portion 6. A filter 101 is mounted on the filter-supporting portion 7 and, at the connecting portion 6, the upper wall 3 is fitted to the upper housing 4, whereby the filter 101 can be mounted so as to cover the to-be-analyzed-gas inlet 2.

To the lower end of the upper housing 4 is fitted a disk-shaped bottom housing 5. The upper housing 4 and the bottom housing 5 constitute a housing 8 of sensor 100.

A detection portion 10 is constituted by a detection element 12 for carbon monoxide gas, a ceramic substrate 14 having a heater (not shown) at the lower side, and support tubes 16 and 18 for detection portion. The detection element 12 is provided on the upper side of the ceramic substrate 14. The ceramic substrate 14 is supported inside the housing 8 by the support tubes 16 and 18 for detection portion which perforate through the bottom housing 5. Into the support tubes 16 and 18 for detection portion are inserted a wire connecting the detection element 12 and an ammeter (not shown) and a wire (not shown) connecting the heater and an external electric source (not shown) for heater.

In the detection portion 10, the detection element is heated to an appropriate operating temperature (200°C to 300°C) by the heater provided at the lower side of the ceramic substrate 14. The change of resistance of the detection element 12 controlled in the above temperature range is measured electrically, whereby the concentration of the carbon monoxide which comes in contact with the surface of the detection element 12, is detected.

A to-be-analyzed gas passes through the to-be-analyzed-gas inlet 2, then permeates the filter 101 by diffusion, and reaches the surface of the detection element 12. NOx and SOx are adsorbed by the filter 101 when the to-be-analyzed gas permeates the filter 101; consequently, the amounts of NOx and SOx reaching the surface of the detection element 12 are substantially zero. Meanwhile, carbon monoxide is not substantially adsorbed by the filter 101. As a result, the detection element 12 detects carbon monoxide without being substantially affected by NOx and SOx.

As the detection element 12, there can be used, with no restriction, a known P type metal oxide semiconductor composed mainly of copper oxide. An example of the semiconductor is a sintered material composed mainly of CuO and containing a Na compound in an amount of about 0.1 to 15 mass %. It is specifically a sintered material described in JP-A-1999-118747, Japanese Patent No. 3333257 or the like.

The filter 101 provided so as to cover the inlet 2 is made of an inorganic material.

The material for the filter is preferably a silicon oxide (e.g. silica gel), activated clay, a hydrous silicon aluminum (e.g. zeolite), etc. Of these, zeolite is suitable. As the zeolite, a zeolite having a large silicic acid component ratio is preferred. Specifically, a zeolite having a $SiO_2/Al_2O_3$ molar ratio of 5 to 510 is preferred, a zeolite having a $SiO_2/Al_2O_3$ molar ratio of 5 to 300 is more preferred, and a zeolite having a $SiO_2/Al_2O_3$ molar ratio of 5 to 30 is particularly preferred. The filter made of a zeolite having such a component ratio has high heat resistance and causes neither oxidation nor disappearance. Further, such a filter adsorbs and removes the NOx and SOx present in a to-be-analyzed gas, for a practically sufficient period of time (500 hours or more) and prevents contact of the detection element with the gas; as a result, selective detection of carbon monoxide by the detection element is possible for a practically sufficient length of time. Such a zeolite having a large silicic acid component ratio is commercially available.

Next, description is made on the process for production of filter, for a case of using zeolite. Filter production using other inorganic material can be conducted in substantially the same manner as in the case of using zeolite.

A zeolite filter can be produced by press-molding a zeolite into a desired shape to obtain a shaped article. This shaped article can be used per se as a filter without firing it.

In the press-molding of a zeolite, a binder may be added to the molding material as long as the addition does not impair the adsorptivity of the filter obtained. The amount of the binder added is preferably 5 mass % or less, more preferably 1 to 2 mass %. The binder may be a known binder such as polyvinyl alcohol or the like.

The particle size of the zeolite to be press-molded is preferably 200 $\mu$m or less, more preferably 50 to 100 $\mu$m.

When the filter obtained has an insufficient strength owing to its shape, etc., it is preferred that the filter obtained is fired in a temperature range wherein there is no reduction in gas adsorptivity, in order to increase the strength of the filter. It is known that, when a zeolite is fired at high temperatures, the crystal structure changes into a cristobalite type. Change of zeolite structure into cristobalite type results in a reduction in NOx adsorptivity. Therefore, the firing temperature is preferred to be not higher than a temperature at which there is no change in zeolite crystal structure. In zeolite, the temperature at which the crystal structure changes, differs depending upon the composition of zeolite; therefore, the firing temperature of zeolite need be selected appropriately depending upon the composition of zeolite. In general, the firing temperature is preferably 900°C or less, more preferably 800 to 850°C.

When a zeolite is used as a catalyst or as an adsorbent, it is used in an atmosphere of 400°C or above, in some cases. Meanwhile, the adsorptivity of zeolite for NOx and SOx is striking at a lower temperature range (60 to 250°C). Therefore, in detecting the carbon monoxide contained in a to-be-analyzed gas, using the gas sensor of the present invention, it is necessary that the temperature of the gas is kept in the above-mentioned range.

The shape of filter may be determined appropriately depending upon the shape of housing and is not particularly restricted. However, the filter shape is preferably a disc shape of 0.5 to 1.5 mm in thickness and 10 to 15 mm in diameter, in view of the handleability.

As shown in Fig. 1, there is no particular restriction as to the structure of filter 101 as long as it is made of at least an inorganic material (e.g. zeolite). An inorganic cushion material layer may be formed on at least one side of the filter.

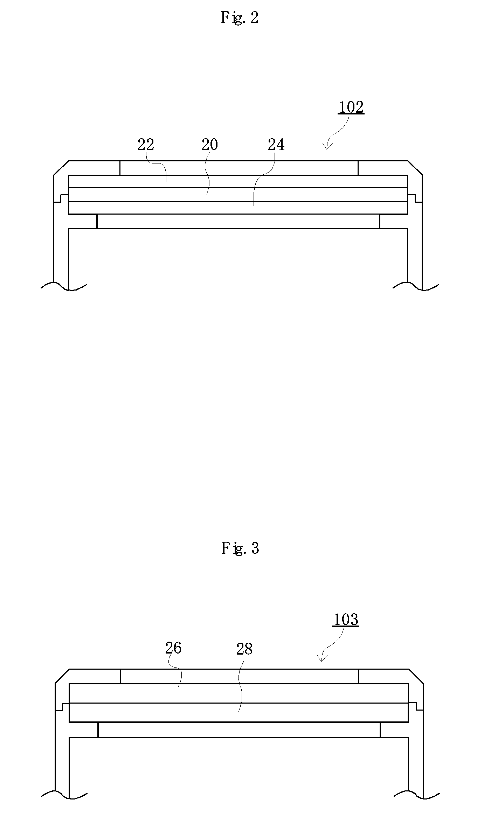

As shown in Fig. 2, the structure of filter 102 is preferably a laminated structure comprising a zeolite layer 20 and inorganic cushion material layers 22 and 24 formed on the both sides of the zeolite layer 20. With this laminated structure, the strength of the zeolite layer can be increased without reducing the adsorptivity for NOx and SOx.

The inorganic cushion material constituting the inorganic cushion material layers 22 and 24 is preferably an inorganic material of good gas permeability and heat resistance. A specific example thereof is an alumina fiber. As a commercial inorganic fiber, Ceramic Paper (a product of Takumi Sangyo) is preferred. The cushion material layers 22 and 24 have each a thickness of preferably 0.4 to 1 mm.

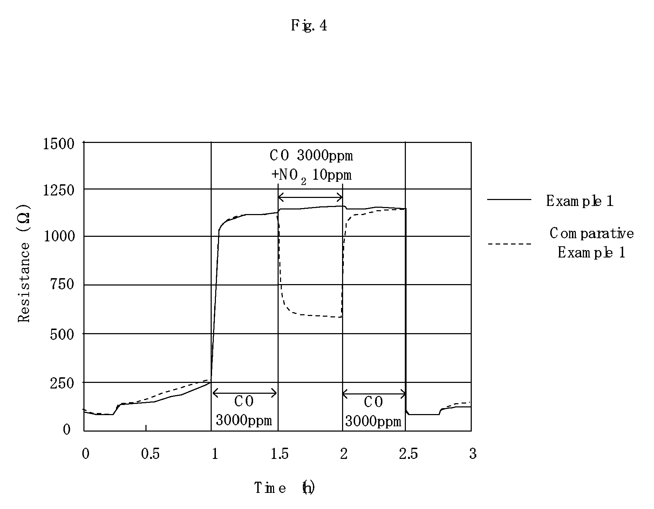

Fig. 3 shows a filter 103 obtained by laminating an active carbon layer 28 on one side of a zeolite layer 26. This filter 103 is fitted in a gas sensor with the zeolite layer 26 being directed toward the outside of a housing 8. By allowing the filter 103 to have such a structure, the filter 103 can have the adsorptivity of the active carbon layer 28 in addition to the adsorptivity of the zeolite layer 26 and thereby can possess a higher removability for the NOx and SOx gases present in a to-be-analyzed gas. In this filter 103, the zeolite layer is fitted with being directed toward the outside of the housing 8; therefore, a to-be-analyzed gas of high temperature makes no direct contact with the active carbon layer. As a result, in this filter 103, there is no reaction of active carbon layer with oxygen (i.e. no oxidation) and no disappearance of active carbon layer over a long period of time even when the gas to be analyzed contains oxygen. There is no particular restriction as to the thickness of the active carbon layer but the thickness is preferably 1 to 2 mm.

The filter is preferably coated with a water-proof agent in order to allow the filter to have a higher moisture resistance.

The temperature of to-be-analyzed gas is ordinarily higher than 100°C during the period in which a combustion apparatus fitted with the sensor of the present invention is in an ordinary combustion operation. Therefore, in this period, there is no condensation of the steam contained in the to-be-analyzed gas. However, there is a period (e.g. a period of idling operation) in which a fuel is burnt in an amount smaller than in the period of ordinary operation. In this period, the combustion amount of fuel is smaller; the temperature of to-be-analyzed gas is lower and may be about 60°C; during this period, the steam contained in the to-be-analyzed gas may cause condensation.

When the steam contained in the to-be-analyzed gas condenses on the filter of the sensor, the adsorptivity of the filter is impaired and the adsorptivity for SOx and NOx drops. As a consequence, accurate detection of carbon monoxide becomes impossible. When a water-proof agent is coated on the filter, the pores of the adsorbent (e.g. zeolite) constituting the filter are not plugged even when there is condensation of steam on the filter. Accordingly, there is no decrease in the adsorptivity and removability of filter.

The water-proof agent is exposed to a to-be-analyzed gas. Therefore, the water-proof agent is preferred to have a heat resistance of 250°C or above and further cause no disappearance by oxidation. As such a water-proof agent, there is desired one which is made of an inorganic material and contains no organic substance and through which condensed water does not permeate but a gas permeates.

As specific examples of the water-proof agents, there can be mentioned, for example, a colloidal silica which is a spherical silica gel having particle diameters of 0.1 $\mu$ or less, preferably 30 to 10 nm, and alumina [e.g. AEROSIL (trade name)]. As the method for coating the water-proof agent, there can be mentioned a method of immersing a filter in a dispersion containing about 0.1 mass % of the water-proof agent and then drying the filter.

As a commercial water-proof agent, there can be mentioned NANOCOAT (a colloidal silica, a product of Nikkosha).

Examples

Example 1

A gas sensor having a structure shown in Fig. 1 was fitted in a passage of a to-be-analyzed gas. The detection element of the gas sensor was a P type semiconductor (thickness: 1 mm, width: 1.5 mm, length: 1.5 mm) composed mainly of CuO and containing 2 mass % of Na element, obtained by firing at 700°C.

The filter fitted to the sensor was produced by adding a polyvinyl alcohol (as a sintering agent) to a commercial zeolite (F-9, a product of Tosoh Corporation, 75 $\mu$m or less in particle diameter), press-molding the mixture at a pressure of 20 MPa, and firing the press molding at 800°C for 15 minutes. The obtained filter had a disc shape of 2 mm in thickness and 10 mm in diameter.

While the temperature of the detection element was being controlled at 250°C, a base gas and two kinds of to-be-analyzed gases (all the gases had a temperature of 200°C) (details of the gases are described later) were fed into the passage in order, to measure the change of the resistance of the detection element. The flow rate of the to-be-analyzed gas was 1 l/min.

The base gas was composed mainly of nitrogen gas and contained carbon dioxide in an amount of 7.5 volume % and oxygen in an amount of 7.5 volume %. The base gas was allowed to contain steam in an amount equivalent to saturated steam at 90°C. This base gas simulates an exhaust gas discharged from the exhaust gas outlet of a gas combustion apparatus of normal combustion state.

One of the to-be-analyzed gases, i.e. the to-be-analyzed gas 1 was a mixture of the base gas and 3,000 ppm of carbon monoxide, and the other, i.e. the to-be-analyzed gas 2 was a mixture of the base gas, 3,000 ppm of carbon monoxide and 10 ppm of nitrogen dioxide.

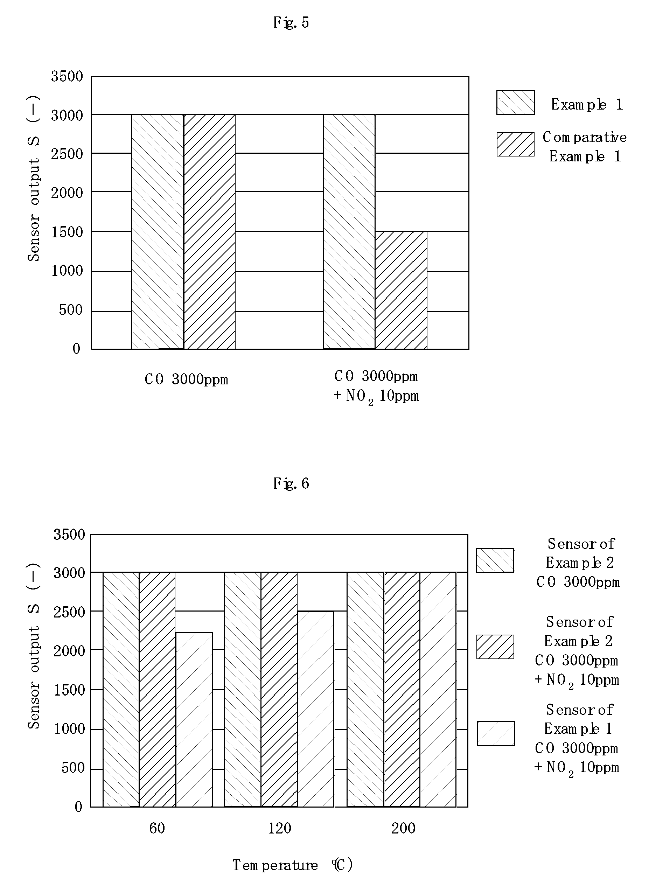

The measurement results of Example 1 are shown in Fig. 4 and Fig. 5. Fig. 4 is a graph showing the change with time, of the measured resistance of the detection element. Fig. 5 is a graph showing the effect of nitrogen dioxide on the output of the detection element.

In Fig. 5, the sensor output S was calculated using the following expression (1).

$$S = 3000 \times R_{NO2}/R_{3000} \qquad (1)$$

In the above expression, $R_{3000}$ is a resistance of detection element shown when the detection element was exposed to the to-be-analyzed gas 1 (which was a mixture of the base gas and 3,000 ppm of carbon monoxide), and $R_{NO2}$ is a resistance of detection element shown when the detection element was exposed to the to-be-analyzed gas 2 (which was a mixture of the base gas, 3,000 ppm of carbon monoxide and 10 ppm of $NO_2$).

This sensor output S was calculated by defining the resistance of sensor shown when the detection element was exposed to the to-be-analyzed gas (which was a mixture of the base gas and 3,000 ppm of carbon monoxide), as sensor output 3,000 and then proportionally calculating a sensor output at that time, from the resistance of sensor shown when the detection element was exposed to the to-be-analyzed gas (which was a mixture of the base gas, 3,000 ppm of carbon monoxide and 10 ppm of nitrogen dioxide).

Comparative Example 1

The change of the resistance of detection element was measured in the same manner as in Example 1 except that the filter of the gas sensor was removed. The measurement results of Comparative Example 1 are shown in Fig. 4 and Fig. 5.

As shown in Fig. 4 and Fig. 5, the resistance and sensor output S of detection element showed a large reduction in the sensor of Comparative Example 1, when the to-be-analyzed gas contained nitrogen dioxide (a period of 1.5 to 2 hours). However, in the detection element of Example 1, the resistance and sensor output S were hardly affected by nitrogen dioxide.

Example 2

A commercial zeolite (F-9, a product of Tosoh Corporation) was press-molded and fired at 800°C for 15 minutes to obtain a filter. This filter was immersed in an aqueous solution containing 10 mass % of a water-proof agent [NANOCOAT (trade name of a Nikkosha product)] and then dried at 150°C. The resulting filter was fitted to the same sensor as used in Example 1. The sensor output S was determined by conducting the same operation as in Example 1 except that the temperature of the base gas or each to-be-analyzed gas was changed to 60°C, 120°C and 200°C.

The results of Example 2 are shown in Fig. 6. The sensor output S shown when nitrogen dioxide was mixed, was equivalent to the level shown when carbon monoxide alone was mixed, at any temperature. That is, the effect of dew condensation on sensor was not seen even at 60°C.

The same operation as in Example 2 was conducted for the sensor produced in Example 1, to determine the output of the sensor. The results are shown in Fig. 6. In the sensor of Example 1 whose filter was not coated with the water-proof agent (NANOCOAT), there was a tendency that the output decreased slightly when the temperature of to-be-analyzed gas decreased.

Example 3

Using a gas sensor having a structure shown in Fig. 1, there was conducted an actual apparatus durability test using a water heater (the maximum temperature of exhaust gas was 250°C). As the filter of the gas sensor, there was used a filter obtained by press-molding a commercial zeolite (F-9, a product of Tosoh Corporation), firing the molding at 800°C for 15 minutes, and laminating, on the inner side of the resulting filter, an active carbon layer made of an active carbon fiber, i.e. an active carbon filter (1.5 mm in thickness and 0.02 g in mass). When the combustion time of the water heater passed 1,000 hours, the sensor was removed from the water heater, and the same operation as in Example 1 was conducted to measure the resistance of the detection element.

Incidentally, by conducting an actual apparatus durability test under the same conditions as above except that the active carbon filter had been removed, it had been confirmed that the time enabling continuous adsorption for $NO_2$ is 744 hours when using the zeolite filter alone.

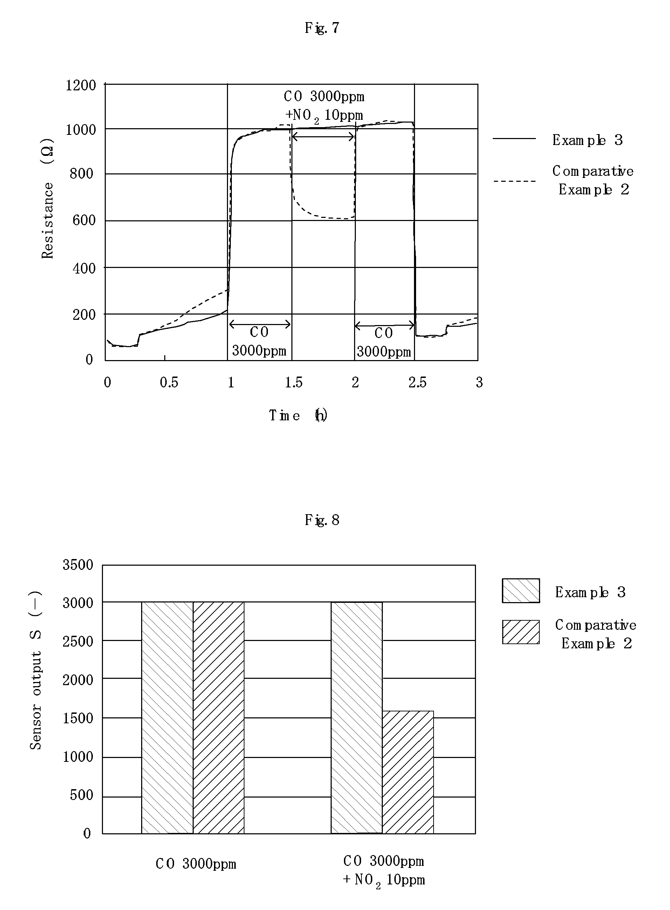

There were shown, in Fig. 7, the change with time, of the resistance of the detection element measured in Example 3 and, in Fig. 8, the sensor output S.

Comparative Example 2

An actual apparatus durability test was conducted in the same manner as in Example 3 except that there was used a gas sensor provided only with the active carbon fiber filter used in Example 3 (using no zeolite filter). Then, the same operation as in Example 1 was conducted to measure the resistance of the detection element.

There were shown, in Fig. 7, the change with time, of the resistance of the detection element measured in Comparative Example 2 and, in Fig. 8, the sensor output S.

As to the active carbon fiber filter used in Example 3 and Comparative Example 2, it is known that, in an actual apparatus durability test (maximum temperature: 250°C) using a water heater, the filter causes oxidation and disappearance when the combustion time has passed 300 hours, and occurring reduced $NO_2$ adsorptivity.

From Fig. 7 and Fig. 8 it has become clear that, in the sensor used in Example 3, the active carbon fiber filter shows no disappearance caused by oxidation and has high $NO_x$ adsorptivity even when the actual apparatus durability test has passed 1,000 hours.

Example 4

The sensor used in Comparative Example 1 (the sensor obtained by removing, from the sensor used in Example 1, the filter) was exposed to a base gas and a base gas containing 6 ppm of $SO_2$ under the same conditions as in Example 1, to measure the resistance Rbase to the base gas and the resistance Rgas to the $SO_2$-containing gas.

The gas sensitivity defined by the following expression (2) was 3.6. Thus, in the gas sensor containing no filter, the resistance varies greatly by the presence of $SO_2$.

$$\text{Gas sensitivity} = \text{Rgas/Rbase} \qquad (2)$$

Next, the gas sensor used in Example 1 (the sensor had a filter) was exposed to a base gas containing 1,000 ppm of CO, in the same manner as in Example 1.

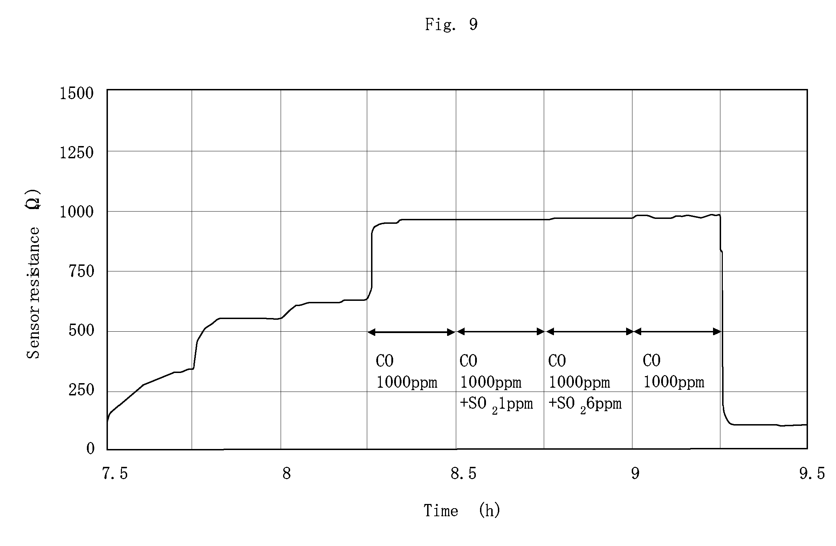

1 ppm of $SO_2$ and then 6 ppm of $SO_2$ were added to the base gas. However, as shown in Fig. 9, there was no variation in sensor resistance.

**Claims**

1. A carbon monoxide sensor comprising
   a housing having a to-be-analyzed-gas inlet fitted with a filter for removal of nitrogen oxides and sulfur oxides, and
   a detection element provided in the housing, which comprises a P type metal oxide semiconductor composed mainly of copper oxide,
   wherein the filter is made of an inorganic material.

2. A carbon monoxide sensor according to Claim 1, wherein the inorganic material is a zeolite having a compositional ratio $SiO_2/Al_2O_3$ (mole basis) of 5 to 510.

3. A carbon monoxide sensor according to Claim 1, wherein the filter is made of a non-fired zeolite.

4. A carbon monoxide sensor according to Claim 1, wherein the filter is obtained by press molding of a non-fired zeolite.

5. A carbon monoxide sensor according to Claim 1, wherein the filter is obtained by firing of a zeolite having an average particle diameter of 150 $\mu$m or less.

6. A carbon monoxide sensor according to Claim 1, wherein the filter is formed in a disc shape.

7. A carbon monoxide sensor according to Claim 1, wherein the filter has a water-proof agent coated thereon.

8. A carbon monoxide sensor according to Claim 1, wherein the filter has a laminated structure of a zeolite layer and an active carbon layer and is fitted to the to-be-analyzed-gas inlet with the zeolite layer being directed toward the outside of the housing.

**9.** A carbon monoxide sensor according to Claim 1, wherein the filter has an inorganic cushion material layer on at least one side of the filter.

**10.** A carbon monoxide sensor according to Claim 9, wherein the inorganic cushion material layer is made of an alumina fiber.

**11.** A carbon monoxide sensor according to Claim 9, wherein the cushion material layer has a thickness of 0.4 to 1 mm.

**12.** A method for detection of carbon monoxide, which comprises detecting a to-be-analyzed gas of 60 to 250°C using a carbon monoxide sensor set forth in Claim 1.

Fig. 1

100

2    3

b ----- b

4a

4

(a)

100

2    101    3

7    6

14    12

4    10

5

4a

8    16    18

(b)

Fig. 2

Fig. 3

Fig. 4

Fig. 5

Fig. 6

Fig. 7

Fig. 8

EP 1 720 008 A1

Fig. 9

Time (h)

## INTERNATIONAL SEARCH REPORT

| | International application No. |
|---|---|
| | PCT/JP2005/003411 |

**A. CLASSIFICATION OF SUBJECT MATTER**
Int.Cl⁷  G01N27/12

According to International Patent Classification (IPC) or to both national classification and IPC

**B. FIELDS SEARCHED**

Minimum documentation searched (classification system followed by classification symbols)
Int.Cl⁷  G01N27/12

Documentation searched other than minimum documentation to the extent that such documents are included in the fields searched
Jitsuyo Shinan Koho          1922-1996    Jitsuyo Shinan Toroku Koho    1996-2005
Kokai Jitsuyo Shinan Koho     1971-2005    Toroku Jitsuyo Shinan Koho    1994-2005

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | JP 10-123083 A  (Chichibu Onoda Cement Corp.),<br>15 May, 1998 (15.05.98),<br>Par. Nos. [0011], [0029] to [0035]<br>(Family: none) | 1<br>3-6,12<br>2,7-11 |
| Y | JP 6-186198 A  (Tokuyama Corp.),<br>08 July, 1994 (08.07.94),<br>Par. No. [0026]; Fig. 1<br>(Family: none) | 3-6,12 |
| Y | JP 1-227951 A  (Figaro Engineering Inc.),<br>12 September, 1989 (12.09.89),<br>Page 5, upper right column, line 11 to<br>lower left column, line 13<br>(Family: none) | 5 |

☐  Further documents are listed in the continuation of Box C.    ☐  See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>31 May, 2005 (31.05.05) | Date of mailing of the international search report<br>21 June, 2005 (21.06.05) |
|---|---|
| Name and mailing address of the ISA/<br>Japanese Patent Office | Authorized officer |
| Facsimile No. | Telephone No. |

Form PCT/ISA/210 (second sheet) (January 2004)

**REFERENCES CITED IN THE DESCRIPTION**

**Patent documents cited in the description**

- JP 3333257 B
- JP 2000338072 A
- JP 11118747 A